# EUROPEAN PATENT APPLICATION

(11) **EP 3 669 929 A1**
(43) Date of publication of application: **24.06.2020**
(21) Application number: 18845645.3
(22) Date of filing: 17.08.2018
(51) Int. Cl.: A61M 37/00

(54) **MICRONEEDLE ARRAY FOR LIPS**

(30) Priority: 17.08.2017 JP 2017157681
(71) Applicant: Cosmed Pharmaceutical Co., Ltd., Kyoto-shi, Kyoto 601-8014 (JP)
(72) Inventor: QUAN, Ying-shu, Kyoto-shi Kyoto 601-8014 (JP); KAMIYAMA,Fumio, Kyoto-shi Kyoto 601-8014 (JP); TANAKA, Hiroshi, Kyoto-shi Kyoto 601-8014 (JP); KONDO, Naoko, Kyoto-shi Kyoto 601-8014 (JP)
(74) Representative: Ter Meer Steinmeister & Partner
(86) International application number: PCT/JP2018/030587
(87) International publication number: WO 2019/035489

(57) **Abstract**

The invention provides a composition that more effectively and simply realizes lightening and plumping of lips. Provided is a microneedle array for lips applied locally to lips in order to lighten and/or plump the lips, the microneedle array including a water-soluble polymer, in which a microneedle has a height of 50 µm to 300 µm, and a tip of the microneedle is a circle having a diameter of 5 to 150 µm or a plane having the same area.

## Description

### TECHNICAL FIELD

The present invention relates to the technical field of microneedles locally applied to lips, and specifically relates to lip lightening and/or lip plumping technique.

### BACKGROUND ART

There is a strong interest in lightening lip pigmentation. Common skin symptoms include hyperpigmentation and spots. In addition, "thinning" of the lips due to aging or fatigue is a matter that recently attracts a lot of interest among women.

Many substances have been used to lighten the skin. For example, hydroquinone, kojic acid, licorice and/or its derivative, ascorbic acid/ascorbic acid derivative, arbutin and the like are used. It has been known to use them as lip balm for lightening the lips as valuable materials.

On the other hand, techniques of cosmetic surgeons such as hyaluronic acid injection have been frequently used against thinning of the lips.

Microneedle preparations have high transdermal absorbability, and development of cosmetic products and pharmaceutical agents has been attempted. In general, an application site of the microneedle preparation is the skin epidermis, but for example, a microneedle patch for vaccination by intrabuccal administration is known (Patent Document 1). This microneedle patch is designed to penetrate an outer layer of the intrabuccal mucous membrane. A microneedle including a microneedle-shaped biocompatible matrix and porous particles on the surface or inside thereof is known (Patent Document 2) . This microneedle may be used under the eyes, on a lower lip, or on cheek wrinkles, and is formed into a size and shape applicable to an application site such as under the eyes and around the mouth.

It is known that the microneedle preparations may administer cosmetic products and pharmaceutical agents by extending the epidermis to reduce a protective characteristic of the epidermis without protrusions thereof penetrating into a keratinous layer (Patent Document 3). This administration device further includes a microprotrusion at a tip of the protrusion.

### PRIOR ART DOCUMENTS

### PATENT DOCUMENTS

Patent Document 1: JP 2015-515474 A
Patent Document 2: JP 2016-87474 A
Patent Document 3: JP 2007-130417 A

### SUMMARY OF THE INVENTION

### PROBLEM TO BE SOLVED BY THE INVENTION

An object of the present invention is to provide a composition that lightens and plumps lips more effectively and simply.

### MEANS FOR SOLVING THE PROBLEM

A technique of using a microneedle for intrabuccal administration, or under the eyes or on the lower lip has been published, but the lips have no keratin and intradermal tissue thereof is covered with an outer mucous membrane. Insertion of microneedles into the lip to break the mucous membrane and deliver a valuable material into the skin should be avoided as this causes intradermal irritation and inflammation. As a result of intensive studies in view of the specialty of the lip tissue, the present inventors have achieved the invention of a microneedle array suitable for the lips by forming the microneedle itself and the substrate itself on which the microneedle stands of a relatively soft substance having a lower elastic modulus than that of metal and silica and allow the same to adhere to the lips, and further finely adjusting a height thereof such that this does not break the lip outer mucous membrane and remains in the outer mucous membrane.

The present invention is as follows.
[1] A microneedle array for lips applied locally to lips in order to lighten and/or plump the lips, the microneedle array including: a water-soluble polymer, in which a microneedle has a height of 50 µm to 300 µm.
[2] The microneedle array for lips according to [1] , in which a tip of the microneedle is a circle having a diameter of 5 to 150 µm or a plane having the same area.
[3] The microneedle array for lips according to [1] or [2], in which density of microneedles is 50 to 2,000 pieces/cm².
[4] The microneedle array for lips according to any one of [1] to [3], in which a thickness of a substrate of the microneedle is 3 to 200 µm.
[5] The microneedle array for lips according to any one of [1] to [4], in which the water-soluble polymer is hyaluronic acid or its derivative.
[6] The microneedle array for lips according to any one of [1] to [5], in which the microneedle array is lined with a protective adhesive tape, a base of the adhesive tape is water-permeable, and an adhesive is partially applied.
[7] The microneedle array for lips according to any one of [1] to [6], in which a microneedle part contains one or more of a lightening ingredient, a moisturizing ingredient, and an anti-inflammatory ingredient.
[8] The microneedle array for lips according to any one of [1] to [7], further including: a pigment or a synthetic colorant.
[9] The microneedle array for lips according to any one of [1] to [8], in which two microneedle arrays are held on one sheet to be applied simultaneously to upper and lower lips.

### EFFECT OF THE INVENTION

A surprising and unexpected fact has been found that local lightening compositions or plumping compositions exert high efficacy that was previously impossible. A specific strategy useful for the composition and method according to the present invention has been provided by applying a microneedle made of a water-soluble material to the lips. The microneedle is a well-known drug transdermal absorbing means. However, before the present inventors, there is no report predicting that the microneedle applied to the lips is extremely effective for lightening and plumping, and no report of trying this. It may be considered that the reason is as follows. That is, the microneedle is originated in research and development in the United States, but microneedle materials there are mainly very hard materials such as stainless steel, silica, titanium, and engineering plastics; there is a sense of discomfort in applying the microneedle of such materials to soft and bending lips.

In a case where the microneedle array of the present invention is applied to the lips, since the lips do not have a keratinous layer, penetration of valuable materials is faster than that in the skin in general, and therefore, an effect may be exerted even in a short time application.

### BRIEF DESCRIPTION OF THE DRAWINGS

FIG. 1 is a cross-sectional view illustrating an example of a method of manufacturing a microneedle array of the present invention.
FIG. 2 is a plan view of a microneedle array sheet manufactured in an Example 2.

### MODE FOR CARRYING OUT THE INVENTION

A characteristic of a microneedle array according to the present invention is a nature of a material (being water soluble) and a needle shape.

### Needle shape of microneedle array

Lips have no keratin and intradermal tissue thereof is covered with an outer mucous membrane. The lips are prominent organs, and insertion of microneedles to break the mucous membrane and deliver a valuable material into the skin should be avoided as this causes intradermal irritation and inflammation. A shape and height of the microneedle must be strictly controlled and designed to prevent the insertion into the skin. Specifically, a tip of the needle is desirably a circle having a diameter of 5 µm or more or a plane having the same area. When a tip area is less than 5 µm, there is a risk of breaking through the lip to be inserted into the skin when being applied. The needle shape is desirably not a bar shape but a truncated cone shape or a conide. In a case of the truncated cone shape or the conide shape, when a needle part is applied to the lip, a contact area with the lip outer mucous membrane is large and the lip is compressed, so that the lip outer mucous membrane is pulled to be thin, which is advantageous for intradermal delivery of the valuable material. On the other hand, in a case of the bar-shaped needle, the contact area with the lip outer mucous membrane is small, which is disadvantageous for delivery of the valuable material. A diameter of the needle tip is desirably 150 µm or less. When the tip diameter exceeds 150 µm, an effect of pulling the lip outer mucous membrane is reduced and a function as the microneedle is lost.

The needle height is desirably 50 µm or more and 300 µm or less, and more preferably 100 µm or more and 250 µm or less. When this is less than 50 µm, a degree of compression on the lip is small, which is disadvantageous for the delivery of the valuable material. When this exceeds 300 µm, there is a risk that the needle breaks through the outer mucous membrane when being applied.

Needle density is desirably 50 to 2,000 pieces/cm², and more preferably 100 to 1200 pieces/cm². When this is 50 pieces/cm² or less, a content of the valuable materials is small, and a delivery amount of the valuable materials is insufficient. When this is 2,000 pieces/cm² or more, it becomes difficult to insert into the soft lip mucous membrane.

Furthermore, in order to apply the needle to the lip mucous membrane to dissolve the same quickly, a design of a thickness of a needle substrate is also important. The thickness of the substrate is desirably 3 to 200 µm, and more preferably 5 to 50 µm. When this is 3 µm or less, toughness of film formation is weak and it is difficult to support the needle exactly. When this is 200 µm or more, the film becomes hard and has difficulty in following a shape of the soft lip, which makes it difficult to apply. In addition, a large amount of water or a long time is required to dissolve the whole including the substrate by externally applying moisture.

### Material of microneedle array

It is important that a material of the microneedle array is a water-soluble material with low hardness. By preparing the microneedle array containing the valuable materials uniformly by using such a material by a conventional method, the valuable materials are included not only in a microneedle part but also in a substrate part. It is also possible to contain the valuable materials only in the microneedle part by a two-step filling manufacturing method. When this microneedle array is applied to the lip, the microneedle part compresses the lip and pulls the lip outer mucous membrane to make the same thin, thereby promoting delivery of the valuable materials contained therein in the lip. When the microneedle array is prepared of a flexible material, the substrate also follows bending of the lip to adhere to the lip, so that the substrate is also compressed and the valuable materials present therein are also delivered into the lip although an amount thereof is relatively smaller than that of the microneedle part.

The material of the microneedle array may include water-soluble polymers such as hyaluronic acid and its derivative (for example, sodium salt, polyethylene oxide grafted hyaluronic acid, hyaluronic acid propylene glycol ester, carboxymethylated sodium hyaluronate, and acetyl sodium hyaluronate), collagen, proteoglycan, hydroxypropyl cellulose, chondroitin sulfate, and carboxymethyl cellulose, and hyaluronic acid or its derivative is preferable. The water-soluble polymers are characterized by swelling when they absorb a small amount of water. The water-soluble polymer dissolved in the lip mucous membrane through the microneedle absorbs water and swells, and brings about an effect of lifting vertical wrinkles of the lips and moistening the lips.

Hyaluronic acid is a type of glycosaminoglycan (mucopolysaccharide) and has a structure in which disaccharide units of N-acetylglucosamine and glucuronic acid are linked. Examples of hyaluronic acid include living organism-derived hyaluronic acid isolated from cockscombs, umbilical cords and the like, culture-derived hyaluronic acid mass-produced by lactic acid bacteria, streptococci and the like, for example. From living organism-derived hyaluronic acid, collagen of the living organism from which this is derived cannot be completely removed, and remaining collagen might have an adverse effect, so that culture-derived hyaluronic acid that does not contain collagen is preferred. Therefore, hyaluronic acid preferably contains 50% by weight or more of culture-derived hyaluronic acid.

When preparing the microneedle array using water-soluble polymer substances selected from hyaluronic acid or its derivative as ingredients, the microneedle array formed from the polymer substances tends to be harder and easily stick in the skin as a weight-average molecular weight thereof decreases, and tends to be softer and hardly stick in the skin as the weight-average molecular weight thereof increases and mechanical strength improves to increase stiffness. For the purpose of the present invention, the weight-average molecular weight is preferably 5,000 to 2,000,000.

When applying the microneedle array to the lip, the microneedle array may be formed of a mixture of high-molecular weight polymer substances having the weight-average molecular weight of 100,000 or more and low-molecular weight polymer substances having the weight-average molecular weight of 50,000 or less in order to make the same hard to such a degree that the outer mucous membrane is not pierced and the microneedle array is hardly broken, and make the valuable materials to be easily penetrated. The weight-average molecular weight of the high-molecular weight polymer substances may be 50,000 or more, and preferably 2,000,000 or less. The weight-average molecular weight of the low-molecular weight polymer substances may be 50,000 or less, and preferably 1,000 or more. In the present invention, the weight-average molecular weight is a value measured by gel permeation chromatography (GPC).

A ratio when the high-molecular weight polymer substances and the low-molecular weight polymer substances are mixed varies depending on the type and weight-average molecular weight of each polymer substance, so that this may be appropriately determined so as to obtain preferable mechanical strength and hardness; however, in general, this is preferably 1% by weight or more of the high-molecular weight polymer substances and 99% by weight or less of the low-molecular weight polymer substances.

### Valuable material

In the microneedle array of the present invention, the material and needle shape described above are important and novel, but well-known materials may be used as the valuable material with which the microneedle is impregnated. In order to achieve lip lightening, substances effective for melanin synthetase inhibition such as hydroquinone, kojic acid, licorice and/or its derivative, ascorbic acid/ethylascorbic acid, ascorbic acid derivative such as ascorbic acid glucoside, and arbutin may be added. In addition, antioxidants such as astaxanthin, fullerene, and coenzyme Q10, blood flow promoters such as vitamin E, ingredients that promote turnover such as retinol, retinal, and adenosine, ingredients that improve metabolism such as niacinamide, dipotassium glycyrrhizinate for rough lip prevention, and fragrance normally used as cosmetic products may be added. In addition, moisturizing ingredients such as ceramide, vitamins A and E, and urea, and anti-inflammatory ingredients such as dipotassium glycyrrhizinate may be added.

An additive amount varies and depends on the type and nature of the substance and the degree of effect desired. Lightening agents and other additives are generally present by an amount from about 0.001 wt% to about 20 wt%, more preferably from about 0.01 wt% to about 5 wt%, and most preferably from about 0.1 wt% to about 2.5 wt% based on the total weight of a composition.

In order to further advance from the lightening of the lips and make the lips to have fresher colors, a composition in which a lipstick ingredient is added to the microneedle as the valuable material may be used after the lightning or simultaneously with the lightening. An additive amount thereof corresponds to that of a lightening agent. Commonly used pigments made of natural dyes such as safflower and cochineal, and dyes (colorants) that are synthetic colorants may be preferably used. As the synthetic colorants, Red No. 104, Red No. 105, Red No. 201, Red No. 203, Red No. 205, Red No. 206, Red No. 207, Red No. 208 and the like may be preferably used.

In order to plump the lips, the polymer substances such as hyaluronic acid and its derivative, collagen, chondroitin sulfate, proteoglycan, and placenta that swell in the skin are used as the valuable materials. Among them, hyaluronic acid and its derivative are suitable as the materials of the microneedle, so that in order to plump the lips, this may be achieved by microneedle molding with hyaluronic acid alone. That is, in this case, it may be said that hyaluronic acid serves as both the material and valuable material of the microneedle array.

The method of manufacturing the microneedle array of the present invention is not especially limited, and this may be manufactured by any conventionally known method; for example, there is a method of casting an aqueous solution of the above-described high-molecular weight hyaluronic acid and low-molecular weight hyaluronic acid, and valuable materials as needed on a mold on which a shape of the microneedle is bored and peeling the same after drying. In a case where the valuable material is contained only in the microneedle part, a manufacturing method in which a valuable material-containing raw material and a valuable material-free raw material are filled at two steps (two-step filling method) may be used. After being peeled, this is cut into a lip shape and lined with a protective adhesive tape to be used. In order to quickly dissolve the needle part of the microneedle array applied to the lip on the lip, it is effective to supply water or serum from the back of the array. It is also effective to use the protective adhesive tape having low water vapor permeability in order to prevent moisture loss from the lips and to steam the microneedle array to promote needle dissolution. In a case where a base of the lining protective adhesive tape of the microneedle array is water-permeable (for example, non-woven fabric), partial application of an adhesive makes it easy to supply water with gauze and the like, and by massaging a microneedle array application part thereafter, it is easy to achieve fast dissolution.

In order to dissolve fast, it is effective to use a readily soluble high-molecular material such as hydrolyzed hyaluronic acid or hydrolyzed collagen in addition to the polymer substances described above. Moreover, dissolubility of the needle is promoted by the composition containing 10% or more of water-soluble substances having a molecular weight of 1,000 or less, vitamin C, monosaccharides, disaccharides and the like.

Although the microneedle array of the present invention is applied to the lips, since the lips do not have a keratinous layer, penetration of the valuable material is faster than that of the skin in general, and therefore, an effect may be exerted even with a short time application.

A local composition (microneedle array) according to the present invention may cope with various skin conditions such as aging spots, dark spots, hyperpigmentation, post-inflammation hyperpigmentation (for example, post-decubitus hyperpigmentation), and discoloration.

The microneedle array of the present invention may optionally include one or more of the following ingredients: anesthetics, anti-allergenic agents, antifungal agents, anti-inflammatory agents, antiseptics, chelating agents, colorants, emollients, exfollients, film formers, fragrances, moistening agents, insect repellents, lubricants, moisturizers, pharmaceutical agents, preservatives, skin protectants, skin penetration enhancers, stabilizers, surfactants, thickeners, viscosity modifiers, or vitamins.

### EXAMPLES

Hereinafter, the present invention is described with reference to examples; however, the present invention is not limited to the examples.

### Example 1

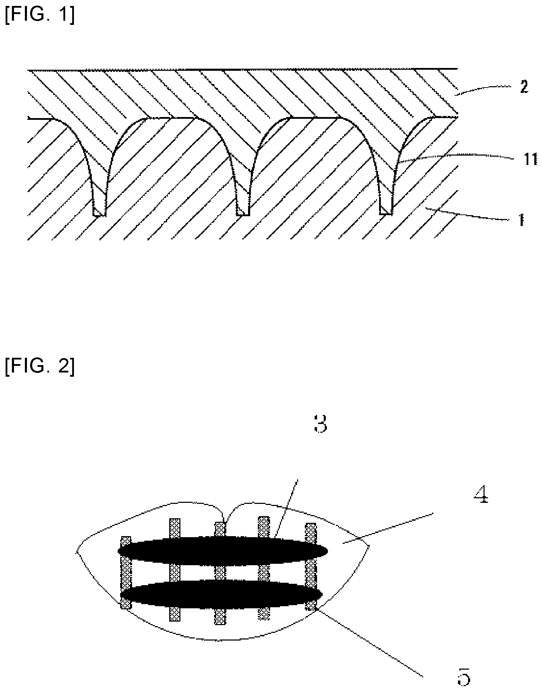

FIG. 1 is a cross-sectional view illustrating an example of a method of manufacturing a microneedle array of the present invention. In the drawing, a reference numeral 1 represents a mold in which a conide type microneedle forming concave portion 11 obtained by forming a conide type microneedle pattern by a lithography method in which a photosensitive resin is irradiated with light and then transferring the conide type microneedle pattern by electroforming is formed.

The microneedle forming concave portion 11 is of a conide type with a root diameter of 0.6 mm, a tip diameter of 0.02 mm, and a depth of 0.2 mm, and the concave portions are arranged in a lattice pattern at intervals of 0.6 mm.

An aqueous solution obtained by dissolving 20 parts by weight of hyaluronic acid (produced by Kikkoman Biochemifa Company, with trade name of "FCH-SU", molecular weight 100, 000) and 0.1 part by weight of Red No. 104 in 100 parts by weight of water at room temperature was cast on the mold 1, this was heated until moisture in a hyaluronic acid aqueous solution layer is evaporated, thereafter this was peeled from the mold 1 and punched into a rounded rectangle (7×50 mm), and the obtained microneedle array was set at the center of a rounded rectangular (9×56 mm) adhesive tape, thereby obtaining the microneedle array of the present invention. A thickness of a substrate of the microneedle array was 60 µm.

The microneedle array of the present invention was pressed together with the adhesive tape against a right side of the lips of two volunteers to be fixed with an adhering part, and was peeled off after 30 minutes. Immediately after this, the lips were briefly washed with water, and the red pigment adhering to the surface was washed away. When the lips were observed in this state, the right side was redder obviously in both the volunteers, and a plump feeling was observed compared to the left side.

### Example 2

A composition is similar to that in the Example 1 except that 2 parts by weight of arbutin was added, and an oval microneedle array having a major axis of 5 cm and a minor axis of 0.8 cm was manufactured using a mold similar to that in the Example 1. The microneedle array of the present invention was obtained by lining the back of the microneedle array with an adhesive tape obtained by pattern applying (vertical strips of 2 mm at interval of 10 mm) of an adhesive to a single surface of non-woven fabric having a basis weight of 50 g/m². Refer to FIG. 2 for specific modes.

Immediately after pressing the microneedle array of the present invention together with the adhesive tape against the upper and lower lips of two volunteers and fixing the same with the adhesive tape, water was supplied from the non-woven fabric side with gauze, then the gauze was removed and a microneedle array application part was massaged, and the microneedle array was peeled off after 15 minutes. Thereafter, when observed in the manner similar to that in the Example 1, the lips were redder and plumped clearly. When this application operation was repeated once a day for seven days, transparency of the application part was observed.

### Example 3

PEG-grafted hyaluronic acid was used instead of hyaluronic acid in the Example 1, and 2% by weight of kojic acid, 1% by weight of vitamin C ethyl, 0.1% by weight of dipotassium glycyrrhizinate, 0.1% by weight of Red No. 218 were added to 100g of aqueous solution, and a microneedle array was manufactured in a manner similar to that in the Example 1. An effect of the present invention was also obtained in the manner similar to that in the Example 1.

### DESCRIPTION OF REFERENCE SYMBOLS

- 1: Mold
- 11: Microneedle forming concave portion
- 2: Hyaluronic acid aqueous solution layer
- 3: Microneedle array sheet
- 4: Lined non-woven sheet
- 5: Adhesive

## Claims

1. A microneedle array for lips applied locally to lips in order to lighten and/or plump the lips, the microneedle array comprising: a water-soluble polymer,
wherein a microneedle has a height of 50 µm to 300 µm.

2. The microneedle array for lips according to claim 1, wherein a tip of the microneedle is a circle having a diameter of 5 to 150 µm or a plane having the same area.

3. The microneedle array for lips according to claim 1 or 2, wherein density of microneedles is 50 to 2,000 pieces/cm².

4. The microneedle array for lips according to any one of claims 1 to 3, wherein a thickness of a substrate of the microneedle is 3 to 200 µm.

5. The microneedle array for lips according to any one of claims 1 to 4, wherein the water-soluble polymer is hyaluronic acid or its derivative.

6. The microneedle array for lips according to any one of claims 1 to 5, wherein the microneedle array is lined with a protective adhesive tape, a base of the adhesive tape is water-permeable, and an adhesive is partially applied.

7. The microneedle array for lips according to any one of claims 1 to 6, wherein a microneedle part contains one or more of a lightening ingredient, a moisturizing ingredient, and an anti-inflammatory ingredient.

8. The microneedle array for lips according to any one of claims 1 to 7, further comprising: a pigment or a synthetic colorant.

9. The microneedle array for lips according to any one of claims 1 to 8, wherein two microneedle arrays are held on one sheet to be applied simultaneously to upper and lower lips.
